# FASCICULE DE BREVET EUROPEEN

(11) **EP 3 601 222 B1**
(45) Date de publication et mention de la délivrance du brevet: **02.06.2021**
(21) Numéro de dépôt: 18714333.4
(22) Date de dépôt: 19.03.2018
(51) Int. Cl.: C07C 309/15

(54) **FLUIDE DE FRACTURATION COMPRENANT UN (CO)POLYMERE D'UNE FORME CRISTALLINE HYDRATEE DE L'ACIDE 2-ACRYLAMIDO-2-METHYLPROPANE SULFONIQUE ET PROCEDE DE FRACTURATION HYDRAULIQUE**
FRACKING-FLUID MIT EINEM (CO)POLYMER EINER HYDRATISIERTEN KRISTALLINEN FORM VON 2-ACRYLAMIDO-2-METHYLPROPAN-SULFONSÄURE UND VERFAHREN ZUM HYDRAULISCHEN FRACKING
FRACTURING FLUID COMPRISING A (CO)POLYMER OF A HYDRATED CRYSTALLINE FORM OF 2-ACRYLAMIDO-2-METHYLPROPANE SULPHONIC ACID AND HYDRAULIC FRACTURING METHOD

(30) Priorité: 20.03.2017 FR 1752288
(43) Date de publication de la demande: 05.02.2020
(73) Titulaire: SPCM SA, 42160 Andrézieux-Bouthéon (FR)
(72) Inventeur: FAVERO, Cédrick, 42160 Andrézieux Bouthéon (FR); KIEFFER, Johann, 42160 Andrézieux Bouthéon (FR)
(74) Mandataire: Cabinet Laurent & Charras
(86) Numéro de dépôt international: PCT/FR2018/050661
(87) Numéro de publication internationale: WO 2018/172684

(56) Documents cités:
- US-A1- 2010 274 048
- US-A1- 2013 255 954

## Description

### DOMAINE DE L'INVENTION

L'invention concerne un nouveau fluide de fracturation comprenant au moins un agent de soutènement et au moins un (co)polymère hydrosoluble préparé à partir de la forme cristalline hydratée de l'acide 2-acrylamido-2-méthylpropane sulfonique et/ou d'au moins un de ses sels.

L'invention concerne aussi un procédé de fracturation hydraulique des réservoirs souterrains d'huile et de gaz non conventionnels utilisant ladite composition.

### ETAT ANTERIEUR DE LA TECHNIQUE

La production d'huile (hydrocarbures) et de gaz contenus dans des réservoirs souterrains non conventionnels se développe depuis plusieurs années et nécessite d'ouvrir des fractures dans le réservoir pour une production économique de l'huile et du gaz.

Dans la suite de la description de l'art antérieur et de l'invention, par «réservoirs souterrains non conventionnels », on désigne des gisements nécessitant des technologies particulières d'extractions car n'existant pas sous forme d'une accumulation dans une roche poreuse et perméable (voir *Les hydrocarbures de roche-mère en France Rapport provisoire* - *CGIET n° 2011-04-G* - *Ministère de l'écologie, du développement durable, des transports et du logement* - *Avril 2011).* Pour le gaz non conventionnel, on peut citer les gaz de schiste (shale gas), les gaz de houille (coal bed methane) ou les gaz de réservoirs compacts (tight gas). Pour l'huile non conventionnelle, on peut citer les huiles lourdes (heavy oil), les huiles de schiste (shale oil) ou les huiles de réservoirs compacts (tight oil). Les réserves contenues dans les réservoirs non conventionnels sont énormes et extrêmement étendues dans des zones autrefois inexploitables comme les hydrocarbures de roche-mère tels que les schistes argileux, les gaz de réservoir compact, et les gaz de houille. Aux Etats-Unis, les gaz de schiste sont largement exploités et représentent aujourd'hui 46% du total du gaz naturel produit aux Etats-Unis alors qu'ils ne représentaient que 28% en 1998. Les bassins très étendus sont connus sous le nom de Barnett Shale, Ville Fayette Shale, Mowry Shale, Marcellus Shale, Utica Shale.

Le document US2013/255954 décrit un fluide de fracturation comprenant un polymère soluble préparé à partir de monomères comme par exemple l'acide 2-acrylamido-2-méthylpropane sulfonique. L'exploitation des réservoirs compacts a été rendue possible par une évolution des techniques de forages.

Les techniques de production ont en effet évolué des puits verticaux vers des puits horizontaux, réduisant le nombre de puits de production nécessaires et leur empreinte au sol et permettant de mieux couvrir le volume du réservoir pour en récupérer au maximum le gaz ou l'huile. Cependant, les perméabilités sont insuffisantes pour que l'hydrocarbure migre de la roche mère vers le puits facilement, et ainsi permettre de produire économiquement et en quantité le gaz ou l'huile. Il est donc nécessaire d'augmenter la perméabilité et les surfaces de production par des opérations de stimulation et en particulier par fracturation hydraulique de la roche en contact avec le puits.

### Fracturation hydraulique

La fracturation hydraulique a pour but de créer une perméabilité supplémentaire et engendrer des surfaces de production de gaz ou d'huile plus importantes. En effet, la faible perméabilité, les barrières naturelles de couches compactes et l'imperméabilisation par les opérations de forage limitent fortement la production. Le gaz ou l'huile contenu dans le réservoir non conventionnel ne peut migrer facilement de la roche vers le puits sans stimulation.

Ces opérations de fracturation hydraulique sur les puits horizontaux ont commencé en 1960 dans les Appalaches et, aujourd'hui, plusieurs dizaines de milliers d'opérations ont eu lieues aux Etats-Unis.

Les technologies d'étude, de modélisation du réservoir, de forage, de cimentation et de stimulation sont devenues de plus en plus sophistiquées et mettent en œuvre des équipements permettant d'effectuer ces opérations dans des temps de plus en plus courts avec une analyse précise des résultats.

### La stimulation du réservoir par fracturation hydraulique

Ces opérations consistent à injecter de l'eau à haute pression et à très fort débit de manière à créer des fractures réparties perpendiculairement aux puits de production. On procède généralement en plusieurs étapes afin de créer des fractures sur toute la longueur du puits horizontal, ce qui permet de couvrir un volume maximal du réservoir.

Afin de garder ces fractures ouvertes, on ajoute un agent de soutènement (par exemple du sable, des matières plastiques ou des céramiques calibrées) de manière à empêcher la fermeture de ces fractures et à maintenir la capillarité créée une fois l'injection stoppée.

L'eau seule ne suffit pas à obtenir une bonne efficacité de placement de l'agent de soutènement du fait de sa faible viscosité. Ceci limite sa capacité à maintenir en place l'agent de soutènement dans les fractures. Pour contrer ce problème, on a développé des fluides de fracturation contenant des composés viscosifiants.

Par définition, on dira qu'un composé est viscosifiant lorsqu'il augmente la viscosité des solutions dans lesquelles il est dissous.

En plus d'avoir des propriétés viscosifiantes, le composé doit avoir un profil rhéologique particulier. En effet, le composé doit pouvoir générer une viscosité faible afin de ne pas gêner au transport et au pompage du fluide contenant l'agent de soutènement pendant les forts cisaillements subis lors de l'injection du fluide de fracturation. Une fois injecté, ce même composé doit pouvoir engendrer une viscosité suffisante lorsque le cisaillement diminue pour supporter l'agent de soutènement afin de le maintenir dans les fractures.

Le polymère doit donc apporter des propriétés rhéofluidifiantes à la solution afin d'avoir une viscosité relativement faible lors de l'injection (à cisaillement élevé) et une viscosité forte afin de maintenir l'agent de soutènement en suspension au niveau de la fracture lorsque le cisaillement diminue.

Les propriétés viscoélastiques des polymères en solution sont également à prendre en considération. Cette viscoélasticité, et son importance dans l'application, est décrite dans le document SPE 147206 *(Fracturing Fluid Comprised of Components Sourced Solely from the Food Industry Provides Superior Proppant Transport* - *David Loveless, Jeremy Holtsclaw, Rajesh Saini, Phil Harris, and Jeff Fleming, SPE, Halliburton*) à travers des observations visuelles dans des expériences statiques ou dynamiques, ou encore par des mesures de rhéologie, telle que la mesure des modules visqueux et élastiques (G' et G"), ou la mesure sur rhéomètre de la viscosité en fonction du cisaillement. Ainsi, des propriétés élastiques seront avantageuses pour assurer le transport et la suspension de l'agent de soutènement de la fracture.

Le choix du polymère n'est donc pas évident et nécessite une étude rhéologique approfondie afin d'obtenir des résultats satisfaisants.

Parmi les composés viscosifiants de solutions aqueuses appartenant à l'état de la technique, on peut citer les substances naturelles telle que les gommes guars et leurs dérivés tel que l'hydroxypropylguar (HPG), ou la carboxyméthylhydroxypropyl guar (CMHPG) ; les dérivés cellulosiques tels que la carboxyméthyl cellulose ou l'hydroxyéthyl cellulose. Ces composés sont notamment décrits dans les brevets US4033415, US3888312 et US4801389. Dans le document SPE 152596 *(Hydraulic Fracturing 101: What Every Representative, Environmentalist, Regulator, Reporter, Investor, University Researcher, Neighbor and Engineer Should Know About Estimating Frac Risk and Improving Frac Performance in Unconventional Gas and Oil Wells* - *George E. King, Apache Corporation),* les dernières avancées relatives aux performances des fluides de fracturation sont discutées en détail.

Cependant ces substances naturelles, et en particulier les dérivés de guar, sont aussi utiles dans d'autres applications, comme l'industrie alimentaire ou le textile, et l'essor de l'exploitation des ressources d'huile et de gaz non conventionnels concurrencent ces autres applications. Ceci crée une pression sur la disponibilité de ces produits et engendrent des problèmes de prix.

D'autres composés issus de la pétrochimie peuvent avoir des propriétés viscosifiantes. On peut citer les polymères synthétiques. Les poly(méth)acrylamides, éventuellement partiellement hydrolyses, et les poly(méth)acrylatcs et leurs copolymères sont particulièrement connus. Ces polymères développent une viscosité grâce à leur masse molaire et aux répulsions ioniques interchaînes. Ces polymères sont décrits dans les brevets GB951147, US3727689, US3841402 ou encore US3938594. Le mécanisme régissant la viscosité est lié à une hausse du volume hydrodynamique grâce à des répulsions intra-chaînes, des enchevêtrements inter-chaînes, etc.

Cependant, en présence de forte salinité ou d'une température d'utilisation élevée, ces polymères ne développent pas de forts enchevêtrements et répulsions, ce qui se traduit par une diminution forte de leur pouvoir viscosifiant surtout après avoir subi le cisaillement de l'étape de pompage. Par ailleurs, ces polymères ne présentent généralement pas de propriétés viscoélastiques suffisantes pour supporter l'agent de soutènement dans la fracture. Il faut augmenter le dosage de ces polymères à des niveaux trop élevés pour obtenir les propriétés de suspension de l'agent de soutènement. Les niveaux de dosages ne sont pas viables économiquement.

Les polymères utilisés pour avoir des propriétés viscosifiantes peuvent avantageusement être aussi des réducteurs de frottement permettant de réduire la perte de charge en milieu turbulent et augmenter fortement le débit à puissance et diamètre de tuyau identique.

Les polymères synthétiques à base d'acide 2-acrylamido-2-méthylpropane sulfonique et/ou de ses sels présentent des propriétés de réduction de friction en solution aqueuse intéressantes. Ces polymères sont aussi connus pour leur résistance au cisaillement et à la dégradation thermique, notamment en solutions salées. Toutefois, l'obtention de polymère de très haut poids moléculaire à base d'acide 2-acrylamido-2-méthylpropane sulfonique est difficile sans compter que ces polymères présentent des problèmes de solubilité lorsque leur poids moléculaire augmente. Or, pour avoir un phénomène de réduction de friction optimal et une forte génération de viscosité, il est primordial que le polymère soit dissous rapidement en particulier en solution saline et qu'il ait un très haut poids moléculaire.

### EXPOSE DE L'INVENTION

La Demanderesse a trouvé et mis au point un fluide de fracturation qui permet d'avoir un effet de réduction de friction très élevé tout en garantissant un effet viscosifiant amélioré en solution saline (saumure) ou non saline (eau).

De plus, la bonne solubilité du polymère du fluide de fracturation combiné à son caractère anionique sulfoné permet d'éviter son adsorption sur la roche, ce qui induit un regain de conductivité et donc une augmentation du rendement de production d'huile (hydrocarbures) et de gaz. L'huile (ou les huiles) issue d'une formation souterraine est également appelée pétrole. Il s'agit généralement d'un mélange d'hydrocarbures.

Un premier aspect de l'invention concerne un fluide de fracturation comprenant au moins un agent de soutènement et au moins un (co)polymère hydrosoluble préparé à partir de la forme cristalline hydratée de l'acide 2-acrylamido-2-méthylpropane sulfonique et/ou d'au moins un de ses sels.

Un second aspect de l'invention concerne un procédé de fabrication d'un fluide de fracturation avec au moins un (co)polymère hydrosoluble préparé à partir de la forme cristalline hydratée de l'acide 2-acrylamido-2-méthylpropane sulfonique et/ou d'au moins un de ses sels.

Un troisième aspect de l'invention concerne un procédé de fracturation hydraulique d'un réservoir souterrain d'huile ou de gaz non conventionnel utilisant le fluide de fracturation selon l'invention.

Un quatrième aspect de l'invention concerne un procédé de réduction de friction utilisant un fluide de fracturation dans une opération de fracturation hydraulique d'un réservoir souterrain d'huile ou de gaz non conventionnel utilisant le fluide de fracturation selon l'invention.

Par définition un (co)polymère hydrosoluble est un (co)polymère qui donne une solution aqueuse sans particules insolubles lorsqu'il est dissous sous agitation à 25°C et avec une concentration de 50 g.L⁻¹ dans l'eau.

L'agent de soutènement peut être choisi de façon non restrictive parmi le sable, la céramique, la bauxite, les billes de verre, et le sable imprégné de résine. Il représente préférentiellement de 0,5 à 40%, plus préférentiellement de 1 à 25% et encore plus préférentiellement de 1,5 à 20%, en poids du fluide de fracturation.

Le fluide de fracturation selon l'invention est de préférence obtenu à partir d'entre 0,001% et 1% en poids de (co)polymère hydrosoluble selon l'invention, préférentiellement entre 0,002% et 0,2%, en poids du fluide de fracturation.

Le fluide de fracturation peut comprendre d'autres composés connus de l'homme de l'art, comme ceux cités dans le document SPE 152596, par exemple :
- Des agents anti-gonflement des argiles comme le chlorure de potassium, ou le chlorure de choline, et/ou
- Des biocides pour éviter le développement de bactéries en particulier sulfato réductrices pouvant former des masses visqueuses réduisant les surfaces de passage. On peut citer, par exemple, le glutaraldéhyde, qui est le plus utilisé, ou encore le formaldéhyde ou les isothiazolinones, et/ou
- Des réducteurs d'oxygène comme le bisulfite d'ammonium pour éviter la destruction des autres composants par oxydation et la corrosion des tubes d'injection, et/ou
- Des additifs anticorrosion pour protéger les tubes contre l'oxydation par les quantités résiduelles d'oxygène, le N,N dimethylformamide étant privilégié, et/ou
- Des lubrifiants comme les distillats d'huile, et/ou
- Des chélatants pour le fer comme l'acide citrique, l'EDTA (éthylène diamine tétra-acétique), les phosphonates, et/ou
- Des produits antitartres comme les phosphates, les phosphonates, les polyacrylates ou l'éthylène glycol.

La forme cristalline hydratée de l'acide 2-acrylamido-2-méthylpropane sulfonique a un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta. L'incertitude de ces pics est généralement de l'ordre de 0.1°.

La cristallographie aux rayons X, radiocristallographie ou diffractométrie de rayons X est une technique d'analyse qui permet d'étudier la structure de la matière cristalline à l'échelle atomique. Elle s'appuie sur le phénomène physique de diffraction des rayons X. Un diffractomètre ayant une source au cuivre peut être utilisé.

Une poudre formée d'une phase cristalline particulière va toujours donner lieu à des pics de diffraction dans les mêmes directions. Ce diagramme de diffraction forme ainsi une véritable signature de la phase cristalline. Il est donc possible de déterminer la nature de chaque phase cristalline au sein d'un mélange ou d'un produit pur.

Cette signature est spécifique à chaque composé cristallin organique ou inorganique, et se présente sous la forme d'une liste de pics de position en angle 2θ (2-thêta).

Cette technique est utilisée pour caractériser la matière, en particulier les différentes formes cristallines qui peuvent exister pour une même molécule chimique.

La forme cristalline hydratée de l'acide 2-acrylamido-2-méthylpropane sulfonique a un spectre infra-rouge à transformée de Fourier comprenant des pics à 3280cm⁻¹, 3126cm⁻¹, 1657cm⁻¹, 1595cm⁻¹, 1453cm⁻¹, 1395cm⁻¹, 1307cm⁻¹, 1205cm⁻¹, 1164cm⁻¹, 1113cm⁻¹, 1041cm⁻¹, 968cm⁻¹, 885cm⁻¹, 815cm⁻¹, 794cm⁻¹. L'incertitude de ces pics est généralement de l'ordre de 8cm⁻¹. De manière avantageuse, il s'agit du spectre solide obtenu conventionnellement dans un sel tel que le KBr.

La spectroscopie infra-rouge à transformée de Fourier est l'analyse des vibrations émises, absorbées ou diffusées par les molécules. Cette technique est sensible aux interactions dites courtes (influence de la maille unitaire sur les liaisons). Dans la majorité des cas, les spectres infra-rouges à transformée de Fourier de différents systèmes cristallins diffèrent de manière significative. Le spectre infra-rouge à transformée de Fourier reflète donc les détails de la structure cristalline d'un composé organique.

De manière générale, et sauf indication contraire, le diagramme de diffraction des rayons X et le spectre infra-rouge sont obtenus à 20°C et à une pression de 1 atmosphère (101 325 Pa).

La forme cristalline hydratée de l'acide 2-acrylamido-2-méthylpropane sulfonique a une énergie minimale d'inflammation supérieure à 400 mJ, préférentiellement supérieure à 500 mJ (1 mJ = 10⁻³ joule).

L'énergie minimale d'inflammation représente l'énergie minimale qui doit être fourni à un composé pour provoquer une inflammation. L'énergie peut être électrique ou thermique. L'énergie minimale d'inflammation est une donnée essentielle pour la prise en compte du risque d'explosion lors de la manipulation du produit (transfert, stockage, réaction, mise en forme...).

L'énergie minimale d'inflammation dépend des propriétés de la poudre (composition) ainsi que de sa structure macromoléculaire (granulométrie, forme cristalline, surface spécifique).

Dans le cadre des solides, cette énergie est l'énergie minimale d'une étincelle électrique susceptible d'enflammer un nuage de poussière. Plus la valeur de l'énergie minimale d'inflammation est élevée, moins le solide présente un risque lors de son utilisation, manipulation, stockage.

La mesure de l'énergie minimale d'inflammation est réalisée selon la norme NF EN 13821.

La forme cristalline hydratée de l'acide 2-acrylamido-2-méthylpropane sulfonique présente 4 phénomènes thermiques avec la technique de calorimétrie différentielle à balayage, à 70°C, 100°C, 150°C et 190°C. L'incertitude relative à l'observation de ces phénomènes est généralement de l'ordre de 10°C, avantageusement 5°C ou moins.

Les phénomènes thermiques sont mesurés par calorimétrie différentielle à balayage (DSC). Cette technique exploite la mesure de la variation de chaleur associée à la dénaturation thermique du composé lorsque celui-ci est chauffé à vitesse constante, par exemple avec une rampe de chauffe de 10°C/minute.

Il est généralement reconnu que le phénomène thermique se présentant à 190°C (+/-10°C) est lié au point de fusion de l'acide 2-acrylamido-2-méthylpropane sulfonique.

Selon un mode particulier de l'invention, le (co)polymère hydrosoluble est obtenu au moins à partir d'acide 2-acrylamido-2-méthylpropane sulfonique et/ou d'au moins un de ses sels, 50mol% à 100mol% de l'acide 2-acrylamido-2-méthylpropane sulfonique étant avantageusement sous la forme cristalline hydratée et/ou au moins un de ses sels, plus avantageusement 70 à 100mol%, et encore plus avantageusement 100mol%.

Le (co)polymère hydrosoluble est avantageusement obtenu à partir d'entre 1 et 100 mol% d'acide 2-acrylamido-2-méthylpropane sulfonique, préférentiellement entre 2 et 60 mol% d'acide 2-acrylamido-2-méthylpropane sulfonique, encore plus préférentiellement entre 5 et 30 mol% d'acide 2-acrylamido-2-méthylpropane sulfonique, encore plus préférentiellement entre 5 et 15 mol% d'acide 2-acrylamido-2-méthylpropane sulfonique ; 50mol% à 100 mol% de l'acide 2-acrylamido-2-méthylpropane sulfonique étant avantageusement sous la forme cristalline hydratée et/ou au moins un de ses sels, plus avantageusement 70 à 100mol%, et encore plus avantageusement 100mol%.

De manière générale, l'homme du métier saura adapter la quantité des éventuels monomères additionnels (anionique et/ou cationique et/ou zwitterionique) listés ci-après pour atteindre 100mol%.

De manière générale, sauf indication contraire, par « acide 2-acrylamido-2-méthylpropane sulfonique sous forme cristalline hydratée » on désigne la forme acide et/ou la forme salifiée. Il en est de même pour les monomères anioniques qui peuvent désigner les formes acides et/ou salifiées comme, par exemple, pour l'acide acrylique.

Selon un mode préféré de l'invention, le (co)polymère de l'invention est obtenu à partir de la forme saline de l'acide 2-acrylamido-2-méthylpropane sulfonique sous forme cristalline hydratée. L'acide 2-acrylamido-2-méthylpropane sulfonique est donc de préférence partiellement ou totalement salifié avant polymérisation. La forme acide d'un monomère peut être salifiée avant et/ou pendant et/ou après la (co)polymérisation du ou des monomères.

La forme saline est avantageusement obtenue à partir d'un composé choisi parmi un hydroxyde de métal alcalin ou alcalino-terreux, un oxyde de métal alcalin ou alcalino-terreux, l'ammoniaque, une amine de formule suivante NR₁R₂R₃ (R₁, R₂ et R₃ étant avantageusement des groupements hydrocarbonés, notamment des alkyles) ou un carbonate de métal alcalin ou alcalino-terreux. Un métal alcalin préféré est le sodium.

Le (co)polymère hydrosoluble est, de préférence, obtenu à partir de la forme cristalline hydratée de l'acide 2-acrylamido-2-méthylpropane sulfonique et/ou d'au moins un de ses sels, et d'au moins un monomère non ionique, et/ou au moins un monomère anionique, et/ou au moins un monomère cationique et/ou au moins un monomère zwittérionique.

Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide, le N,N-diéthylacrylamide et le N-méthylolacrylamide. Egalement, peuvent être utilisés la N-vinylformamide, le N-vinyl acétamide, la N-vinylpyridine et la N-vinylpyrrolidone, le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprenol. Un monomère non ionique préféré est l'acrylamide.

Selon un mode de réalisation particulier, le (co)polymère hydrosoluble est avantageusement obtenu à partir de la forme cristalline hydratée de l'acide 2-acrylamido-2-méthylpropane sulfonique ou d'au moins un de ses sels et à partir d'entre 1 et 99 mol% de monomère(s) non-ionique(s), de préférence entre 40 et 95 mol% et plus préférentiellement entre 45 et 90 mol%, par rapport au nombre total de monomères. Dans ce cas, le copolymère est avantageusement obtenu à partir d'entre 1 et 99 mol% d'acide 2-acrylamido-2-méthylpropane sulfonique et/ou au moins un de ses sels, et plus préférentiellement entre 2 et 60 mol% ; 50mol% à 100mol% de l'acide 2-acrylamido-2-méthylpropane sulfonique étant avantageusement sous la forme cristalline hydratée et/ou au moins un de ses sels, plus avantageusement 70 à 100mol%, et encore plus avantageusement 100mol%.

Le ou les monomères anioniques pouvant être utilisés dans le cadre de l'invention peuvent être choisis dans un large groupe. Ces monomères peuvent présenter des fonctions acryliques, vinyliques, maléiques, fumariques, malonique, itaconique, ou allyliques, et contenir un groupe carboxylate, phosphonate, phosphate, sulfate, sulfonate, ou un autre groupe à charge anionique. Le monomère anionique peut être sous forme acide ou bien sous forme de sel de métal alcalino-terreux, de sel de métal alcalin ou de sel d'ammonium. Des exemples de monomères convenables incluent l'acide acrylique ; l'acide méthacrylique ; l'acide itaconique ; l'acide crotonique ; l'acide maléique ; l'acide fumarique ; l'acide acrylamido undécanoïque ; l'acide 3-acrylamido 3-méthylbutanoïque ; l'anhydride maléique ; les monomères de type acide fort, par exemple les monomères présentant une fonction de type acide sulfonique ou acide phosphonique tels que l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylmethacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium. Dans cette liste, les monomères mentionnés de type acide fort présentant une fonction de type acide sulfonique n'incluent pas la forme cristalline hydratée de l'acide 2-acrylamido-2-méthylpropane sulfonique et/ou ses sels.

Selon un mode de réalisation particulier, le copolymère est avantageusement obtenu à partir d'entre 1 et 99 mol% de monomère(s) anionique(s), de préférence entre 2 et 60 mol%, et encore plus préférentiellement entre 3 et 50 mol%, par rapport au nombre total de monomères. Dans ce cas, ces pourcentages incluent également le monomère de forme cristalline hydratée de l'acide 2-acrylamido-2-méthylpropane sulfonique et/ou ses sels.

Le ou les monomères cationiques pouvant être utilisés dans le cadre de l'invention peuvent être choisis, notamment parmi les monomères dérivés des motifs de type acrylamide, acrylique, vinylique, allylique ou maléique, ces monomères possédant une fonction phosphonium ou ammonium quaternaire. On peut citer, en particulier et de façon non limitative, l'acrylatc de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de méthacrylamido propyltriméthyl ammonium (MAPTAC). L'agent de quaternisation peut être choisi parmi les chlorures d'alkyle, les sulfates de dialkyle ou les halogénures d'alkyle. Préférentiellement, l'agent de quaternisation est choisi parmi le chlorure de méthyle ou le diéthyle sulfate.

Les sels acidifiés sont obtenus par les moyens connus de l'homme de métier, et notamment par protonation. Les sels quaternisés sont également obtenus par les moyens connus de l'homme du métier notamment, par réaction avec le chlorure de benzyle, le chlorure de méthyle (MeCl), les chlorures d'aryle, d'alkyle, ou les dialkylsulfates comme le diméthylsulfate.

Selon un mode de réalisation préféré, le monomère cationique est choisi parmi les sels de diallyldialkyl ammonium comme le chlorure de diallyl diméthyl ammonium (DADMAC) ; les sels acidifiés ou quaternisés de dialkyl- aminoalkylacrylamides ou méthacrylamides, comme par exemple le chlorure de méthacrylamido-propyl triméthyl ammonium (MAPTAC), le chlorure d'acrylamido-propyl triméthyl ammonium (APTAC).

Le monomère zwitterionique peut être un dérivé d'un motif de type acrylamide, acrylique, vinylique, allylique ou maléique, ce monomère possédant une fonction amine ou ammonium quaternaire et une fonction acide de type carboxylique (ou carboxylate), sulfonique (ou sulfonate) ou phosphorique (ou phosphate). On peut citer, en particulier et de façon non limitative les dérivés de l'acrylate de diméthylaminoéthyl, tel que le 2-((2-(acryloyloxy)éthyl) diméthylammonio) éthane-1-sulfonate, le 3-((2-(acryloyloxy)éthyl) diméthylammonio) propane-1-sulfonate, le 4-((2-(acryloyloxy)éthyl) diméthylammonio) butane-1-sulfonate, le [2-(acryloyloxy)éthyl] (diméthylammonio) acetate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl] (diméthylammonio) acétate, les dérivés du diméthylamino propylacrylamide tel que le 2-((3-acrylamidopropyl) diméthylammonio) éthane-1-sulfonate, le 3-((3-acrylamidopropyl) diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

Les monomères présentant un caractère hydrophobe peuvent également être utilisés dans la préparation du (co)polymère hydrosoluble utilisé dans le procédé de l'invention. Ils sont de préférence choisis dans le groupe constitué par les esters de l'acide (méth)acrylique présentant une chaîne alkyle, arylalkyle, propoxylée ou éthoxylée ; les dérivés du (méth)acrylamide présentant une chaîne alkyle, arylalkyle ou dialkyle, arylalkyle propoxylée, éthoxylée, éthoxylée et propoxylée, ou dialkyle ; les alkyl aryl sulfonates.

Lorsqu'on utilise un monomère présentant un caractère hydrophobe pour la préparation du (co)polymère hydrosoluble, sa quantité se situe avantageusement dans la plage comprise entre 0,001 et 3 % en mole par rapport à la quantité totale de monomères.

Les monomères présentant une fonction fluorescente peuvent également être utilisés dans le cadre de l'invention. Un monomère présentant une fonction fluorescente peut être détecté par n'importe quelle méthode appropriée, par exemple par fluorométrie avec un fluorimètre à longueur d'onde fixe. Généralement, la détection du monomère présentant une fonction fluorescente se fait aux maxima d'excitation et d'émission, qui peuvent être déterminés à l'aide d'un fluorimètre à balayage.

Les monomères présentant une fonction fluorescente sont choisis, par exemple, parmi les monomères de type styrène sulfonate de sodium ou styrène sulfonique.

Le (co)polymère hydrosoluble est préférentiellement un (co)polymère anionique à base d'acrylamide et d'acide 2-acrylamido-2-méthylpropane sulfonique ; 50mol% à 100mol% de l'acide 2-acrylamido-2-méthylpropane sulfonique étant sous la forme cristalline hydratée et/ou au moins un de ses sels. Préférentiellement, il s'agit d'un terpolymère d'acrylamide, d'acide acrylique et d'acide 2-acrylamido-2-méthylpropane sulfonique ; 50mol% à 100mol% de l'acide 2-acrylamido-2-méthylpropane sulfonique étant sous la forme cristalline hydratée et/ou au moins un de ses sels. Dans les deux cas, le (co)polymère peut être partiellement ou totalement post hydrolysé.

Le (co)polymère hydrosoluble est préférentiellement obtenu à partir d'entre 1% et 99% molaire de monomère(s) anionique(s), plus préférentiellement entre 2% et 60% molaire, ces pourcentages incluant le monomère correspondant à la forme cristalline hydratée de l'acide 2-acrylamido-2-méthylpropane sulfonique ou un de ses sels.

De manière préférée, le (co)polymère hydrosoluble selon l'invention est anionique ou amphotère et est obtenu à partir d'entre 1 et 99 mol% de monomères anioniques ces pourcentages incluant le monomère correspondant à la forme cristalline hydratée de l'acide 2-acrylamido-2-méthylpropane sulfonique ou un de ses sels. Le (co)polymère hydrosoluble selon l'invention est préférentiellement un copolymère d'un sel de la forme cristalline hydratée de l'acide 2-acrylamido-2-méthylpropane sulfonique et d'acrylamide.

Le (co)polymère hydrosoluble selon l'invention est préférentiellement un polymère anionique obtenu par copolymérisation d'un sel de la forme cristalline hydratée de l'acide 2-acrylamido-2-méthylpropane sulfonique avec un sel de l'acide acrylique, ou un polymère anionique obtenu par copolymérisation d'un sel de la forme cristalline hydratée de l'acide 2-acrylamido-2-méthylpropane sulfonique avec un monomère non ionique hydrolysable. Le monomère non ionique hydrolysable est préférentiellement choisi parmi l'acrylamide ; le méthacrylamide ; les dérivés N-mono de l'acrylamide ou du méthacrylamide ; les dérivés N,N de l'acrylamide ou du méthacrylamide ; et les esters acryliques ou méthacryliques. Le monomère non ionique hydrolysable préféré est l'acrylamide.

Selon l'invention, le (co)polymère hydrosoluble peut avoir une structure linéaire, branché, star (en forme d'étoile), comb (en forme de peigne) ou bloc. Ces structures peuvent être obtenues par sélection au choix de l'amorceur, de l'agent de transfert, de la technique de polymérisation telle que la polymérisation radicalaire contrôlée dite RAFT (transfert de chaîne réversible par addition-fragmentation, de l'anglais « reversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais «Atom Transfert Radical Polymerization »), de l'incorporation de monomères structuraux, de la concentration...

Selon l'invention, le (co)polymère hydrosoluble est avantageusement linéaire ou structuré. Par (co)polymère structuré, on désigne un (co)polymère non linéaire qui possède des chaînes latérales de manière à obtenir, lorsque ce (co)polymère est mis en solution dans l'eau, un fort état d'enchevêtrement conduisant à des viscosités à bas gradient très importantes. Le (co)polymère hydrosoluble, selon l'invention n'est généralement pas réticulé.

Le (co)polymère hydrosoluble peut en outre être structuré :
- par au moins un agent de structure, pouvant être choisi dans le groupe comprenant des monomères à insaturation polyéthylénique (ayant au minimum deux fonctions insaturées), comme par exemple les fonctions vinyliques, allyliques, acryliques et époxy et l'on peut citer par exemple le méthylène bis acrylamide (MBA), la triallyamine, le chlorure de tétraallylammonium ou 1,2 dihydroxyethylène bis-(N-acrylamide), et/ou
- par des macroamorceurs tels que les polyperoxydes, polyazoïques et les polyagents de transfert tels que les (co)polymères polymercaptants, et les polyols, et/ou
- par des polysaccharides fonctionnalisés.

La quantité d'agent de ramification/réticulation dans le mélange de monomères est avantageusement inférieure à 4 % en poids par rapport à la teneur en monomères, plus avantageusement inférieure à 1 %, et encore plus avantageusement inférieure à 0,5 %. Selon un mode de réalisation particulier, elle peut être au moins égale à 0.00001 % en poids par rapport à la teneur en monomères.

De manière générale, le (co)polymère ne nécessite pas de développement de procédé de polymérisation particulier. En effet, il peut être obtenu selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; ou de polymérisation micellaire.

La polymérisation est généralement une polymérisation à radicaux libres, de préférence par polymérisation en émulsion inverse ou par polymérisation en gel. Par polymérisation par radicaux libres, nous incluons la polymérisation par radicaux libres au moyen d'initiateurs UV, azoïques, redox ou thermiques ainsi que les techniques de polymérisation radicalaire contrôlée (CRP) ou les techniques de polymérisation sur matrice.

Selon un mode particulier de l'invention, le (co)polymère peut être post hydrolysé. La post hydrolyse est la réaction du (co)polymère après polymérisation. Cette étape consiste en la réaction de groupes fonctionnels hydrolysables de monomères avantageusement non ioniques, plus avantageusement les fonctions amide ou ester, avec un agent d'hydrolyse. Cet agent d'hydrolyse peut être une enzyme, une résine échangeuse d'ion, ou un métal alcalin. Préférentiellement, l'agent d'hydrolyse est une base. Durant cette étape de post-hydrolyse du (co)polymère, le nombre de fonctions acide carboxylique augmente. En effet, la réaction entre la base et les fonctions amides ou esters présentes dans le (co)polymère produit des groupes carboxylates.

Selon l'invention, le (co)polymère peut se présenter sous forme liquide, gel ou solide lorsque sa préparation inclut une étape de séchage tel que le « spray drying » (séchage par pulvérisation), le séchage sur tambour le séchage par rayonnement tel que le séchage micro-ondes, ou encore le séchage en lit fluidisé.

Selon un mode de réalisation particulier, le (co)polymère hydrosoluble peut comprendre au moins un groupe à LCST.

Selon les connaissances générales de l'homme du métier, un groupe à LCST correspond à un groupe dont la solubilité dans l'eau pour une concentration déterminée, est modifiée au-delà d'une certaine température et en fonction de la salinité. Il s'agit d'un groupe présentant une température de transition par chauffage définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition minimale est appelée «LCST» (température critique inférieure de solubilité, de l'anglais «Lower Critical Solution Température »). Pour chaque concentration de groupe à LCST, une température de transition par chauffage est observée. Elle est supérieure à la LCST qui est le point minimum de la courbe. En dessous de cette température, le (co)polymère est soluble dans l'eau, au-dessus de cette température, le (co)polymère perd sa solubilité dans l'eau.

Selon un mode de réalisation particulier, le (co)polymère hydrosoluble peut comprendre au moins un groupe à UCST.

Selon les connaissances générales de l'homme du métier, un groupe à UCST correspond à un groupe dont la solubilité dans l'eau pour une concentration déterminée, est modifiée en dessous d'une certaine température et en fonction de la salinité. Il s'agit d'un groupe présentant une température de transition par refroidissement définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition maximale est appelée «UCST» (température critique supérieure de solubilité, de l'anglais « Upper Critical Solution Température »). Pour chaque concentration de groupe à UCST, une température de transition par refroidissement est observée. Elle est inférieure à la UCST qui est le point maximum de la courbe. Au-dessus de cette température, le (co)polymère est soluble dans l'eau, en-dessous de cette température, le (co)polymère perd sa solubilité dans l'eau.

Selon l'invention, le (co)polymère hydrosoluble a un poids moléculaire avantageusement élevé. Par « poids moléculaire élevé », on désigne des poids moléculaires d'au moins 0,5 million de g/mol, préférentiellement entre 10 et 40 millions g/mol, plus préférentiellement entre 15 et 30 millions g/mol. Le poids moléculaire s'entend en poids moléculaire moyen en poids. Il est mesuré par mesure de la viscosité intrinsèque (formule de Mark-Houwink).

Avant sa mise en œuvre dans le fluide de fracturation, le (co)polymère hydrosoluble selon l'invention peut se trouver sous différentes formes solides ou liquides. Préférentiellement, il se trouve sous forme de poudre, d'émulsion inverse eau dans huile, ou de suspension polyphasique particulaire aqueuse ou huileuse.

Un second aspect de l'invention concerne un procédé de préparation d'un fluide de fracturation avec au moins un (co)polymère hydrosoluble préparé à partir de la forme cristalline hydratée de l'acide 2-acrylamido-2-méthylpropane sulfonique ou d'au moins un de ses sels, dans lequel le (co)polymère est mise en œuvre dans de l'eau ou une saumure, et dans lequel le (co)polymère hydrosoluble est, avant formation du fluide de fracturation :
- Soit sous forme de poudre ;
- Soit sous forme d'une émulsion inverse eau dans huile ;
- Soit sous forme d'une suspension polyphasique particulaire aqueuse ou huileuse.

Le procédé de préparation d'un fluide de fracturation selon l'invention comprend préférentiellement une étape d'ajout dans le fluide d'au moins un agent de soutènement tel que décrit précédemment.

Lorsque le (co)polymère hydrosoluble mis en œuvre dans le fluide de fracturation est, avant formation du fluide de fracturation, sous forme de poudre, la taille des particules est préférentiellement inférieure à 1,5 millimètres, plus préférentiellement inférieure à 850 micromètres, encore plus préférentiellement inférieure à 200 micromètres.

La taille des particules se réfère au diamètre moyen mesuré avec calibreur de particules laser selon les techniques conventionnelles de l'homme de l'art. Un exemple d'appareil pour mesurer le diamètre moyen est le Mastersizer de Malvern Instruments.

Lorsque le (co)polymère hydrosoluble mis en œuvre dans le fluide de fracturation est, avant formation du fluide de fracturation, sous forme d'une émulsion inverse eau dans huile, la concentration en (co)polymère dans l'émulsion est préférentiellement comprise entre 5 et 60 % en poids, plus préférentiellement entre 15 et 40% en poids par rapport au poids de l'émulsion. De manière préférée, l'émulsion inverse eau dans huile contient en poids de 0,01% à 70% d'un sel organique et/ou inorganique, de préférence entre 5 et 20% en poids par rapport au poids de l'émulsion. Les sels peuvent être choisis de façon non restrictive parmi le chlorure de sodium, le sulfate de sodium, le bromure de sodium, le sulfate d'ammonium, le chlorure d'ammonium, le chlorure de lithium, le bromure de lithium, le chlorure de potassium, le bromure de potassium, le sulfate de magnésium, le sulfate d'aluminium et leurs mélanges. Les sels préférés sont le chlorure d'ammonium et le sulfate d'ammonium.

Lorsque le (co)polymère hydrosoluble mis en œuvre dans le fluide de fracturation est, avant formation du fluide de fracturation, sous forme d'une suspension polyphasique particulaire aqueuse, le (co)polymère est de préférence sous la forme d'une suspension. Dans ce cas, le (co)polymère hydrosoluble mis en œuvre dans le fluide de fracturation est, avant formation du fluide de fracturation, sous forme d'une suspension polyphasique particulaire aqueuse comprenant :
i. 15 à 60 % en poids d'au moins un (co)polymère hydrosoluble selon l'invention sous forme de particules solides de taille moyenne comprise entre 5 et 500 µm ;
ii. 15 à 45 % en poids d'au moins un sel de métal alcalin et/ou d'au moins un sel d'un métal alcalino-terreux ;
iii. au moins un agent viscosifiant différent du (co)polymère hydrosoluble selon l'invention ;
iv. au moins 10 % en poids d'eau ; et
ladite suspension ayant une viscosité Brookfield comprise entre 500 et 20000 cps à une température de 20°C, et
ladite suspension ayant une masse volumique comprise entre 1,1 et 2 kg.L⁻¹.

Lorsque le (co)polymère hydrosoluble mis en œuvre dans le fluide de fracturation est sous forme d'une suspension polyphasique particulaire huileuse, ladite suspension comprend de préférence :
i. 15 à 60 % en poids d'au moins un (co)polymère hydrosoluble selon l'invention sous forme de particules solides de taille moyenne comprise entre 5 et 500 µm ;
ii. au moins un agent viscosifiant différent du (co)polymère hydrosoluble selon l'invention ;
iii. au moins 10% en poids d'huile ; et
ladite suspension ayant une viscosité Brookfield comprise entre 500 et 20000 cps à une température de 20°C, et
ladite suspension ayant une masse volumique comprise entre 0,6 et 1,4 kg.L⁻¹.

La viscosité Brookfield est mesurée avec un appareil Brookfield, monté d'un module LV, le module pouvant tourner à une vitesse de 30 tours par minutes par exemple, la mesure étant avantageusement effectuée à 20°C. La masse volumique est mesurée à 20°C, à une pression de 1 atm c'est-à-dire 101 325 Pa.

Un troisième aspect de l'invention concerne un procédé de fracturation hydraulique de réservoir souterrain d'huile ou de gaz non conventionnel comprenant la préparation d'un fluide de fracturation tel que décrit précédemment, et l'injection dudit fluide de fracturation dans une formation souterraine.

L'injection est réalisée sous pression de manière à créer des fractures réparties tout le long du puits de production.

Optionnellement, avant, durant ou après la création des fractures, on injecte dans le réservoir au moins un composé oxydant et/ou au moins un composé tensioactif.

L'injection de tensioactif permet d'éliminer la viscosité engendrée par le (co)polymère en inhibant les interactions hydrophobes interchaînes, tandis que l'injection du composé oxydant détruit le (co)polymère. Dans les deux cas, l'injection permet de rétablir une viscosité de fluide proche de celle de l'eau.

Comme composé oxydant, on peut citer la javel (solutions aqueuse d'un sel d'hypochlorite), l'eau oxygénée, l'ozone, les chloramines, les persulfates, les permanganates ou les perchlorates.

La nature chimique du (ou des) composé(s) tensio-actif(s) n'est pas critique. Ils peuvent être anioniques, non ioniques, amphotères, zwitterioniques et/ou cationiques. De préférence, le(s) composé(s) tensio-actif(s) de l'invention porte(nt) des charges anioniques.

De préférence, les composés tensioactifs utilisés sont choisis parmi les tensio-actifs anioniques et leurs zwitterions choisis dans le groupe comprenant les dérivés d'alkylsulfates, d'alkyléthersulfates, d'arylalkylsulfates, d'arylalkyléthersulfates, d'alkylsulfonates, d'alkyléthersulfonates, d'arylalkylsulfonates, d'arylalkyléthersulfonates, d'alkylphosphates, d'alkyléthérphosphatés, d'arylalkylphosphates, d'arylalkylétherphosphates, d'alkylphosphonates, d'alkylétherphosphonates, arylalkylphosphonates, d'arylalkylétherphosphonates, d'alkylcarboxylates, d'alkyléthercarboxylates, d'arylalkylcarboxylates, d'arylalkyléthercarboxylates, de polyethers alkyles, de polyethers arylalkyles...

On définit par chaine alkyle, une chaine de 6 à 24 carbones, ramifiée ou non, avec plusieurs motifs ou non, pouvant éventuellement comporter un ou plusieurs hétéroatomes (O, N, S). On définit par chaine arylalkyle, une chaine de 6 à 24 carbones, ramifiée ou non, comportant un ou plusieurs noyaux aromatiques et pouvant éventuellement comporter un ou plusieurs hétéroatomes (O, N, S).

Les agents tensio-actifs les plus couramment utilisés, pour des raisons de coût, de stabilité, et de disponibilité, sont du type sulfonate ou sulfate, présentés sous la forme de sels de métaux alcalins ou d'ammonium.

Un quatrième aspect de l'invention concerne un procédé de réduction de friction d'un fluide de fracturation dans une opération de fracturation hydraulique de réservoir souterrain d'huile ou de gaz non conventionnel comprenant la préparation d'un fluide de fracturation tel que décrit précédemment, et l'injection dudit fluide de fracturation dans une formation souterraine.

La réduction de friction permet de diminuer ou de supprimer les pertes liées aux frottements lors de l'injection du fluide de fracturation.

L'invention et les avantages qui en résultent ressortiront bien des exemples de réalisation suivants.

### EXAMPLES

### Synthèse des polymères

### Polymères 1 et 2 (copolymères ATBS/acrylamide post hydrolysés)

### Polymère 1 (ATBS sous forme cristalline hydratée)

Dans un bécher de 2000 mL sont ajoutés 761,9 g d'eau déionisée, 574,2 g d'acrylamide en solution à 50%, 35,9g de lessive de soude à 50%, 11,7g d'urée et 116,3g de cristaux d'acide 2-acrylamido-2-méthylpropane sulfonique (forme cristalline hydratée).

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :
- 0,45 g de 2,2'-azobisisobutyronitrile,
- 1,5 mL d'une solution à 5 g/L de dihydrochlorure de 2,2'-azobis[2-(2-imidazolin-2-yl)propane],
- 1,5 ml d'une solution à 1 g/L d'hypophosphite de sodium,
- 2,25 ml d'une solution à 1 g/L de ter-butyl hydroperoxyde,
- 3,0 ml d'une solution à 1 g/L de sulfate d'ammonium et de fer(II) hexahydraté (sel de Mohr).

Après quelques minutes, l'arrivée d'azote est fermée et le réacteur est clos. La réaction de polymérisation se déroule pendant 2 à 5 heures jusqu'à atteindre un pic de température. Le gel obtenu est haché en particules d'une taille comprise entre 1 et 6 mm.

500,0 g de gel préalablement haché sont ensuite mélangés à 18,0 g de lessive de soude à 50%, le mélange est porté et maintenu à une température de 90°C pour une durée de 90 minutes.

Le gel est ensuite séché et broyé pour obtenir le polymère sous forme de poudre.

### Polymère 2 (ATBS non sous forme cristalline hydratée)

Le polymère 2 est préparé comme le polymère 1 en remplaçant l'acide 2-acrylamido-2-méthylpropane sulfonique de forme cristalline hydratée par de l'acide 2-acrylamido-2-méthylpropane sulfonique n'étant pas de forme cristalline hydratée.

### Polymères 3 et 4 (teryolymères acide acrylique/ATBS/acrylamide)

### Polymère 3 (ATBS sous forme cristalline hydratée)

Dans un bécher de 2000 mL sont ajoutés 542,1g d'eau déionisée, 558,7g d'acrylamide en solution à 50%, 104,8 g de lessive de soude à 50%, 75,5g d'acide acrylique glacial, 15,3 g d'urée et 203,6 g de cristaux d'acidc 2-acrylamido-2-méthylpropane sulfonique

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout

Sont ensuite ajoutés dans le réacteur :
- 1,13 g de 2,2'-azobisisobutyronitrile,
- 1,5 mL d'une solution à 15 g/1 de dihydrochlorure de 2,2'-azobis[2-(2-imidazolin-2-yl)propane],
- 1,5 ml d'une solution à 3 g/L d'hypophosphite de sodium,
- 0,75 ml d'une solution à 1 g/L de ter-butyl hydroperoxyde,
- 2,25 ml d'une solution à 1 g/L de persulfate de sodium,
- 1,5 ml d'une solution à 2 g/L de sulfate d'ammonium et de fer(II) hexahydraté (sel de Mohr).

Après quelques minutes, l'arrivée d'azote est fermée et le réacteur est clos. La réaction de polymérisation se déroule pendant 1 à 5 heures jusqu'à atteindre un pic de température. Le gel obtenu est haché en particules d'une taille comprise entre 1 et 6 mm.

Le gel est ensuite séché et broyé pour obtenir le polymère sous forme de poudre.

### Polymère 4 (ATBS non sous forme cristalline hydratée)

Le polymère 4 est préparé comme le polymère 3 en remplaçant l'acide 2-acrylamido-2-méthylpropane sulfonique de forme cristalline hydratée par de l'acide 2-acrylamido-2-méthylpropane sulfonique n'étant pas de forme cristalline hydratée.

### Polymères 5 et 6 (homopolymères d'ATBS)

### Polymère 5 (ATBS sous forme cristalline hydratée)

Dans un bécher de 2000 mL sont ajoutés 390,5 g d'eau déionisée, 262 g de lessive de soude à 50% et 847,5 g de cristaux d'acide 2-acrylamido-2-méthylpropane sulfonique.

La solution ainsi obtenue est refroidie entre 5 et 10°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :
- 0,45 g de 2,2'-azobisisobutyronitrile,
- 1,5 ml d'une solution à 2.5 g/1 de dihydrochlorure de 2,2'-azobis[2-(2-imidazolin-2-yl)propane],
- 1,5 ml d'une solution à 1 g/l d'hypophosphitc de sodium,
- 1,5 ml d'une solution à 1 g/l de ter-butyl hydroperoxyde,
- 1,5 ml d'une solution à 1 g/l de sulfate d'ammonium et de fer(II) hexahydraté (sel de Mohr).

Après quelques minutes, l'arrivée d'azote est fermée et le réacteur est clos. La réaction de polymérisation se déroule pendant 2 à 5 heures jusqu'à atteindre un pic de température. Le gel obtenu est haché et séché pour obtenir une poudre grossière elle-même broyée et tamisée pour obtenir le polymère sous forme de poudre.

### Polymère 6 (ATBS non sous forme cristalline hydratée)

Le polymère 6 est préparé comme le polymère 5 en remplaçant l'acide 2-acrylamido-2-méthylpropane sulfonique de forme cristalline hydratée par de l'acide 2-acrylamido-2-méthylpropane sulfonique n'étant pas de forme cristalline hydratée.

### Polymères 7 et 8 (copolymères ATBS/acrylamide)

### Polymère 7 (ATBS sous forme cristalline hydratée)

Dans un bécher de 2000 mL sont ajoutés 549,5 g d'eau déionisée, 520,5 g d'acrylamide en solution à 50%, 97,6 g de lessive de soude à 50%, 16,2 g d'urée et 316,2 g de cristaux d'acide 2-acrylamido-2-méthylpropane sulfonique.

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :
- 0,75 g de 2,2'-azobisisobutyronitrile,
- 1,5 ml d'une solution à 5 g/L de dihydrochlorure de 2,2'-azobis[2-(2-imidazolin-2-yl)propane],
- 1,5 ml d'une solution à 3 g/L d'hypophosphite de sodium,
- 2,25 ml d'une solution à 1 g/L de ter-butyl hydroperoxyde,
- 2,25 ml d'une solution à 1 g/L de sulfate d'ammonium et de fer(II) hexahydraté (sel de Mohr).

Après quelques minutes, l'arrivée d'azote est fermée et le réacteur est clos. La réaction de polymérisation se déroule pendant 1 à 5 heures jusqu'à atteindre un pic de température. Le gel obtenu est haché en particules d'une taille comprise entre 1 et 6 mm.

Le gel est ensuite séché et broyé pour obtenir le polymère sous forme de poudre.

### Polymère 8 (ATBS non sous forme cristalline hydratée)

Le polymère 8 est préparé comme le polymère 7 en remplaçant l'acide 2-acrylamido-2-méthylpropane sulfonique de forme cristalline hydratée par de l'acide 2-acrylamido-2-méthylpropane sulfonique n'étant pas de forme cristalline hydratée.

### Exemples 2 : Préparation de fluides de fracturation

Les polymères 1 à 8 sous forme de poudre sont mis en solution sous agitation à une concentration de 10 000ppm dans une saumure composée d'eau, de 85 g de chlorure de sodium (NaCl) et de 33,1 g de chlorure de calcium (CaCl₂, 2H₂O) par litre de saumure. Les solutions salines de polymères ainsi obtenues sont alors injectées à une concentration de 0,05 pptg dans la saumure mise en recirculation pour les tests Flow Loop qui suivent.

### Exemple 3 : Tests de réduction de friction Flow Loop

Pour évaluer la réduction de friction de chacun des polymères 1 à 8 le réservoir de la boucle du Flow Loop a été rempli de 20 L de saumure (saumure décrite dans l'exemple 2) La saumure est alors mise en recirculation dans la boucle du Flow Loop à un débit de 24 gallons par minutes. Le polymère est ajouté à une concentration de 0,5 pptg dans la saumure en recirculation. Le pourcentage de réduction de friction est ainsi déterminé grâce à la mesure de variations de pression mesurée à l'intérieur de la boucle Flow Loop.

### Figures 1 à 4 :

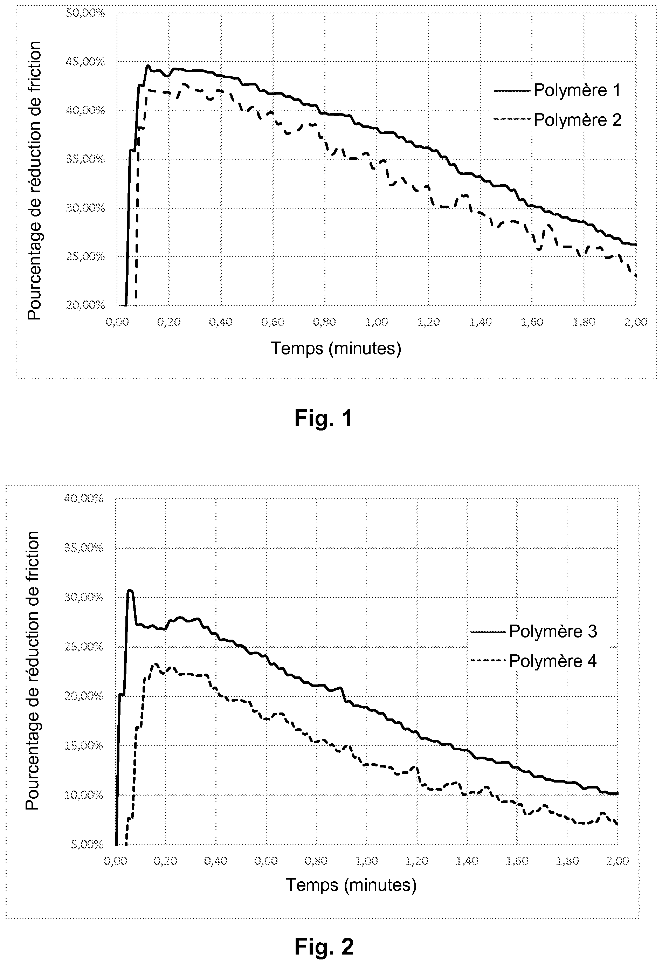

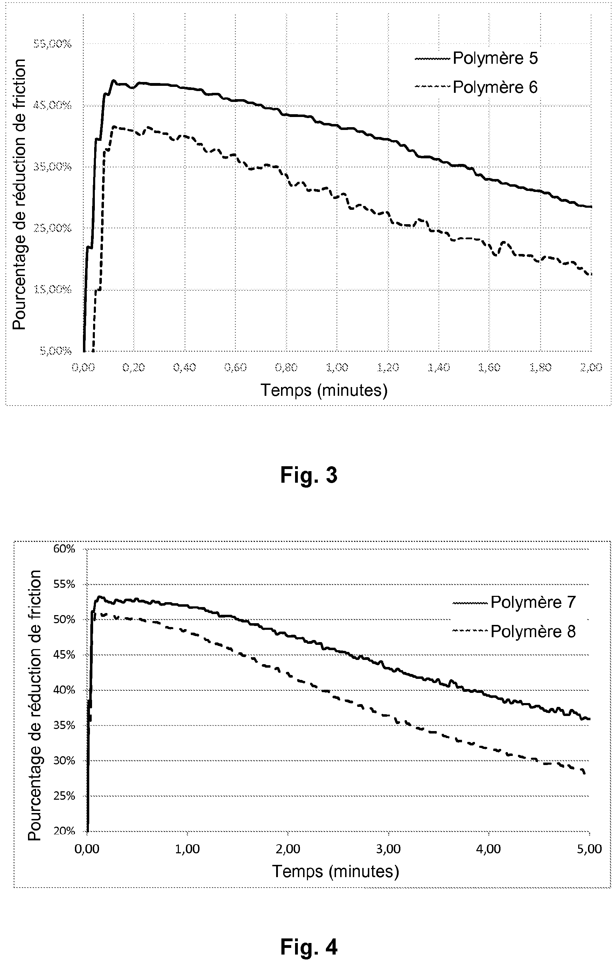

Les figures 1 à 4 sont des graphiques montrant le pourcentage de réduction de friction en fonction du temps pour chaque type de polymère. (Figure 1 : post hydrolysés, figure 2 : terpolymères, figure 3 : copolymères, figure 4 : homopolymères)

Ces figures mettent en évidence que les fluides d'injection selon l'invention permettent une réduction de friction améliorée. En effet, lorsque les polymères contiennent l'ATBS sous forme cristalline hydratée, la réduction de friction est meilleure.

## Revendications

1. Fluide de fracturation comprenant au moins un agent de soutènement et au moins un (co)polymère hydrosoluble préparé à partir de la forme cristalline hydratée de l'acide 2-acrylamido-2-méthylpropane sulfonique et/ou d'au moins un de ses sels,
la forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta.

2. Fluide de fracturation selon la revendication 1, ***caractérisé* en ce que** le (co)polymère hydrosoluble est obtenu au moins à partir d'acide 2-acrylamido-2-méthylpropane sulfonique et/ou d'au moins un de ses sels, 50mol% à 100mol% de l'acide 2-acrylamido-2-méthylpropane sulfonique étant sous la forme cristalline hydratée et/ou au moins un de ses sels.

3. Fluide de fracturation selon l'une des revendications précédentes, ***caractérisé* en ce que** le (co)polymère hydrosoluble est obtenu à partir de la forme cristalline hydratée de l'acide 2-acrylamido-2-méthylpropane sulfonique et/ou d'au moins un de ses sels, et d'au moins un monomère non ionique, et/ou au moins un monomère anionique, et/ou au moins un monomère cationique et/ou au moins un monomère zwittérionique.

4. Fluide de fracturation selon l'une des revendications précédentes, ***caractérisé* en ce que** l'acide 2-acrylamido-2-méthylpropane sulfonique sous forme cristalline hydratée est partiellement ou totalement salifié avant polymérisation.

5. Fluide de fracturation selon l'une des revendications précédentes, ***caractérisé* en ce que** le (co)polymère hydrosoluble est anionique ou amphotère et comprend entre 1 et 99mol% de monomères anioniques.

6. Fluide de fracturation selon l'une des revendications précédentes, ***caractérisé* en ce que** le (co)polymère hydrosoluble est un copolymère d'un sel de la forme cristalline hydratée de l'acide 2-acrylamido-2-méthylpropane sulfonique et d'acrylamide.

7. Fluide de fracturation selon l'une des revendications précédentes, ***caractérisé* en ce que** le (co)polymère hydrosoluble est un polymère anionique obtenu par copolymérisation d'un sel de la forme cristalline hydratée de l'acide 2-acrylamido-2-méthylpropane sulfonique avec un sel de l'acide acrylique, ou un polymère anionique obtenu par copolymérisation d'un sel de la forme cristalline hydratée de l'acide 2-acrylamido-2-méthylpropane sulfonique avec un monomère non ionique hydrolysable.

8. Fluide de fracturation selon l'une des revendications précédentes, ***caractérisé* en ce que** le fluide de fracturation comprend entre 0,001% et 1% en poids de (co)polymère hydrosoluble préparé à partir de la forme cristalline hydratée de l'acide 2-acrylamido-2-méthylpropane sulfonique et/ou d'au moins un de ses sels.

9. Procédé de préparation du fluide de fracturation selon l'une des revendications 1 à 8, par mise en œuvre, dans de l'eau ou une saumure, d'au moins un (co)polymère hydrosoluble préparé à partir de la forme cristalline hydratée de l'acide 2-acrylamido-2-méthylpropane sulfonique et/ou d'au moins un de ses sels, et dans lequel le (co)polymère hydrosoluble est, avant formation du fluide de fracturation :
- soit sous forme de poudre ;
- soit sous forme d'une émulsion inverse eau dans huile ;
- soit sous forme d'une suspension polyphasique particulaire aqueuse ou huileuse.

10. Procédé selon la revendication 9, ***caractérisé* en ce que** le (co)polymère hydrosoluble est, avant formation du fluide de fracturation, sous forme de poudre ayant une taille de particules inférieure à 1,5 millimètres, préférentiellement inférieure à 850 micromètres, encore plus préférentiellement inférieure à 200 micromètres.

11. Procédé selon l'une des revendications 9 ou 10, ***caractérisé* en ce que** le (co)polymère hydrosoluble est, avant formation du fluide de fracturation, sous forme d'une émulsion inverse eau dans huile, la concentration en (co)polymère dans l'émulsion étant comprise entre 5 et 60 % en poids, préférentiellement entre 15 et 40% en poids par rapport au poids de l'émulsion.

12. Procédé selon l'une des revendications 9 à 11, ***caractérisé* en ce que** le (co)polymère hydrosoluble est, avant formation du fluide de fracturation, sous forme d'une émulsion inverse eau dans huile contenant en poids de 0,01% à 70% d'un sel organique et/ou inorganique, de préférence entre 5 et 20% en poids par rapport au poids de l'émulsion.

13. Procédé selon l'une des revendications 9 à 12, ***caractérisé* en ce que** le (co)polymère hydrosoluble est, avant formation du fluide de fracturation, sous forme d'une suspension polyphasique particulaire aqueuse comprenant :
i. 15 à 60 % en poids d'au moins un (co)polymère hydrosoluble sous forme de particules solides de taille moyenne comprise entre 5 µm et 500 µm ;
ii. 15 à 45 % en poids d'au moins un sel de métal alcalin et/ou d'au moins un sel d'un métal alcalino-terreux ;
iii. au moins un agent viscosifiant différent du (co)polymère hydrosoluble ;
iv. au moins 10 % en poids d'eau ;
ladite suspension ayant une viscosité Brookfield comprise entre 500 et 20000 cps à une température de 20°C, et
ladite suspension ayant une masse volumique comprise entre 1,1 et 2 kg.L⁻¹.

14. Procédé de préparation selon l'une des revendications 9 à 13, ***caractérisé* en ce que** le (co)polymère hydrosoluble est, avant formation du fluide de fracturation, sous forme d'une suspension polyphasique particulaire huileuse comprenant :
i. 15 à 60 % en poids d'au moins un (co)polymère hydrosoluble sous forme de particules solides de taille moyenne comprise entre 5 µm et 500 µm ;
ii. au moins un agent viscosifiant différent du (co)polymère hydrosoluble ;
iii. au moins 10 % en poids d'huile ;
ladite suspension ayant une viscosité Brookfield comprise entre 500 et 20000 cps à une température de 20°C, et
ladite suspension ayant une masse volumique comprise entre 0,6 et 1,4 kg.L⁻¹.

15. Procédé de fracturation hydraulique d'un réservoir souterrain d'huile ou de gaz non conventionnel, comprenant la préparation d'un fluide de fracturation selon l'une des revendications 1 à 8, et l'injection dudit fluide de fracturation dans une formation souterraine.

16. Procédé de réduction de friction d'un fluide de fracturation dans une opération de fracturation hydraulique d'un réservoir souterrain d'huile ou de gaz non conventionnel, comprenant la préparation d'un fluide de fracturation selon l'une des revendications 1 à 8, et l'injection dudit fluide de fracturation dans une formation souterraine.

## Patentansprüche

1. Fracking- Fluid mit mindestens einem Stützmittel und mindestens einem wasserlöslichen (Co)Polymer, hergestellt aus der hydratisierten kristallinen Form von 2-Acrylamido-2-methylpropansulfonsäure und/ oder mindestens einem ihrer Salze, die kristalline Form von 2-Acrylamido-2-methylpropansulfonsäure hat dabei ein Röntgen- Pulverdiffraktogramm, das Spitzen bei 10.58°, 11.2°, 12.65°, 13.66°, 16.28°, 18.45°, 20⁰ 20.4⁰ 22.5°, 25.5°, 25.88°, 26.47⁰, 28.52°, 30.28°, 30.8°, 34.09°, 38.19⁰, 40.69⁰, 41.82⁰, 43.74⁰, 46.04° Grad 2-theta aufweist.

2. Fracking- Fluid nach Anspruch 1, ***dadurch gekennzeichnet, dass*** das wasserlösliche (Co)Polymer hergestellt wird aus mindestens 2-Acrylamido-2-methylpropansulfonsäure und/ oder mindestens einem der Salze, 50mol% bis 100mol% 2-Acrylamido- 2-methylpropansulfonsäure haben dabei die hydratisierte kristalline Form und/ oder mindestens eines der Salze

3. Fracking- Fluid nach einem der vorangehenden Ansprüche, ***dadurch gekennzeichnet, dass*** das wasserlösliche (Co)Polymer hergestellt wird aus der hydratisierten kristallinen Form der 2-Acrylamido- 2-methylpropansulfonsäure und/ oder mindestens einem ihrer Salze, und mindestens einem nicht ionischen Monomer und/ oder mindestens einem anionischen Monomer und/ oder mindestens einem kationischen Monomer und/ oder mindestens einem zwitterionischen Monomer.

4. Fracking- Fluid nach einem der vorangehenden Ansprüche, ***dadurch gekennzeichnet, dass*** die 2-Acrylamido- 2-methylpropansulfonsäure in der hydratisierten kristallinen Form vor der Polymerisation teilweise oder vollständig in Salz überführt wurde.

5. Fracking- Fluid nach einem der vorangehenden Ansprüche, ***dadurch gekennzeichnet, dass*** das wasserlösliche (Co)Polymer anionisch oder amphoter ist und zwischen 1 und 99mol% anionischer Monomere enthält.

6. Fracking- Fluid nach einem der vorangehenden Ansprüche, ***dadurch gekennzeichnet, dass*** es sich bei dem wasserlöslichen (Co)Polymer um ein Salz in hydratisierter kristalliner Form der 2-Acrylamido- 2-methylpropansulfonsäure und von Acrylamid handelt.

7. Fracking- Fluid nach einem der vorangehenden Ansprüche, **dadurch gekennzeichnet, dass** es sich bei dem wasserlöslichen (Co)Polymer um ein anionisches Polymer handelt, hergestellt durch Copolymerisation eines Salzes der hydratisierten kristalline Form der 2-Acrylamido- 2-methylpropansulfonsäure mit einem Salz der Acrylsäure oder ein anionisches Polymer, hergestellt durch Copolymerisation eines Salzes der hydratisierten kristalline Form der 2-Acrylamido-2- methylpropansulfonsäure mit einem nicht ionischen, hydrolysierbaren Monomer.

8. Fracking- Fluid nach einem der vorangehenden Ansprüche, **dadurch gekennzeichnet, dass** das Fracking- Fluid zwischen 0,001 und 1 Gewichts-% wasserlösliches (Co)Polymer enthält, hergestellt aus der hydratisierten kristallinen Form de 2-Acrylamido-2-methylpropansulfonsäure und/ oder mindestens einem ihrer Salze.

9. Verfahren zur Herstellung des Fracking- Fluids nach einem der vorangehenden Ansprüche 1 bis 8, durch Einsatz in Wasser oder einer Salzlauge mindestens eines wasserlöslichen (Co)Polymers, hergestellt aus der hydratisierten kristallinen Form der 2-Acrylamido- 2-methylpropansulfonsäure und/ oder mindestens eines ihrer Salze, und bei der das wasserlösliche (Co)Polymer vor der Bildung des Fracking-Fluid:
- entweder pulverfömig ist;
- oder eine inverse Emulsion Wasser in Öl bildet;
- oder die Form einer teilchenförmigen wässrigen oder öligen Suspension hat.

10. Verfahren nach Anspruch 9, ***dadurch gekennzeichnet, dass*** das wasserlösliche (Co)Polymer vor der Bildung des Fracking- Fluids die Form eines Pulvers hat, mit einer Teilchengröße von unter 1,5 Millimeter, vorzugsweise unter 850 Mikrometer, noch besser unter 200 Mikrometer.

11. Verfahren nach einem der Ansprüche 9 oder 10, ***dadurch gekennzeichnet, dass*** das wasserlösliche (Co)Polymer vor der Bildung des Fracking- Fluids, die Form einer inversen Emulsion Wasser in Öl hat, die (Co)polymer- Konzentration in der Emulsion liegt dabei zwischen 5 und 60 % Gewichts-%, vorzugsweise zwischen 15 und 40 Gewichts-% bezogen auf das Gewicht der Emulsion.

12. Verfahren nach einem der Ansprüche 9 bis 11, ***dadurch gekennzeichnet, dass*** das wasserlösliche (Co)Polymer vor der Bildung des Fracking- Fluids, die Form einer inversen Emulsion Wasser in Öl hat, mit einem Gewichtsanteil von 0,01 % bis 70% eines organischen und/oder anorganischen Salzes, vorzugsweise zwischen 5 und 20 Gewichts-% bezogen auf das Gewicht der Emulsion.

13. Verfahren nach einem der Ansprüche 9 bis 12, **dadurch gekennzeichnet, dass** das wasserlösliche (Co)Polymer vor der Bildung des Fracking- Fluids, die Form einer polyphasischen wässrigen Teilchensuspension hat, die enthält:
i. 15 bis 60 Gewichts-% mindestens eines wasserlöslichen (Co)Polymers in Form fester Teilchen, mit einer durchschnittlichen Größe zwischen 5 µm und 500 µm;;
ii. 15 bis 45 Gewichts-% mindestens eines Erdmetallsalzes und/ oder mindestens eines Erdalkalisalzes;
iii. mindestens ein Verdickungsmittel, das sich vom wasserlöslichen (Co)Polymer unterscheidet;
iv. mindestens 10 Gewichts-% Wasser; diese Suspension hat eine Brookfield-Viskosität zwischen 500 und 20000 cps bei einer Temperatur von 20° C, und die Suspension hat eine Dichte zwischen 1,1 und 2 kg.L⁻¹.

14. Herstellungsverfahren nach einem der Ansprüche 9 bis 13, **dadurch gekennzeichnet, dass** das wasserlösliche (Co)Polymer vor der Bildung des Fracking-Fluids, die Form einer polyphasischen wässrigen Teilchensuspension hat, die enthält:
i. 15 bis 60 Gewichts-% mindestens eines wasserlöslichen (Co)Polymers in Form fester Teilchen, mit einer durchschnittlichen Größe zwischen 5 µm und 500 µm;
ii. mindestens ein Verdickungsmittel, das sich vom wasserlöslichen (Co)Polymer unterscheidet;
iii. mindestens 10 Gewichts-% Öl;
diese Suspension hat eine Brookfield- Viskosität zwischen 500 und 20000 cps bei einer Temperatur von 20° C, und
die Suspension hat eine Dichte zwischen 0,6 und 1,4 kg.L⁻¹.

15. Verfahren zum hydraulischen Fracking eines nicht- konventionellen unterirdischen Öl- oder Gaslagers, das die Herstellung eines Fracking- Fluids nach einem der Ansprüche 1 bis 8 enthält und Einspritzen dieses Fracking- Fluids in eine unterirdische Formation.

16. Verfahren zur Verringerung der Reibung eines Fracking- Fluids eines nichtkonventionellen unterirdischen Öl- oder Gaslagers, das die Herstellung eines Fracking- Fluids nach einem der Ansprüche 1 bis 8 enthält und Einspritzen dieses Fracking- Fluids in eine unterirdische Formation.

## Claims

1. A fracturation fluid comprising at least one propping agent and at least one water-soluble (co)polymer prepared from the hydrated crystalline form of 2-acrylamido-2-methylpropane sulfonic acid and/or of at least one of its salts;
the hydrated crystalline form of 2-acrylamido-2-methylpropane sulfonic acid has a 2-theta powder X-ray diffraction diagram comprising peaks at 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrees.

2. The fracturation fluid according to claim 1, ***characterized* in that** the water-soluble (co)polymer is obtained at least from 2-acrylamido-2-methylpropane sulfonic acid and/or from at least one of its salts, 50 mol% to 100 mol% of 2-acrylamido-2-methylpropane sulfonic acid being in the hydrated crystalline form and/or at least from one of its salts.

3. The fracturation fluid according to one of the previous claims, ***characterized* in that** the water-soluble (co)polymer is obtained from the hydrated crystalline form of 2-acrylamido-2-methylpropane sulfonic acid and/or from at least one of its salts, and from at least one nonionic monomer, and/or at least one anionic monomer, and/or at least one cationic monomer and/or at least one zwitterionic monomer.

4. The fracturation fluid according to one of the previous claims, ***characterized* in that** the 2-acrylamido-2-methylpropane sulfonic acid in hydrated crystalline form is partially or totally salified before polymerization.

5. The fracturation fluid according to any of the previous claims, ***characterized* in that** the water-soluble (co)polymer is anionic or amphoteric and comprises between 1 and 99 mol% of anionic monomers.

6. The fracturation fluid according any of the previous claims, ***characterized* in that** the water-soluble (co)polymer is a copolymer of a salt of the hydrated crystalline form of 2-acrylamido-2-methylpropane sulfonic acid and acrylamide.

7. The fracturation fluid according to one of the previous claims, ***characterized* in that** the water-soluble (co)polymer is an anionic polymer obtained by copolymerization of a salt in the hydrated crystalline form of 2-acrylamido-2-methylpropane sulfonic acid with an acrylic acid salt, or an anionic polymer obtained by copolymerization of a salts of 2-acrylamido-2-methylpropane sulfonic acid in hydrated crystalline form with a nonionic hydrolyzable monomer.

8. The fracturation fluid according to one of the previous claims, ***characterized* in that** the fracturation fluid comprises between 0.001% and 1% by weight of water-soluble (co)polymer prepared from the hydrated crystalline form of 2-acrylamido-2-methylpropane sulfonic acid and/or of at least one of its salts.

9. A preparation process for the fracturation fluid according to one of claims 1 to 8, by using, in water or brine, at least one water-soluble (co)polymer prepared from the hydrated crystalline form of 2-acrylamido-2-methylpropane sulfonic acid and/or of at least one of its salts, and in which the water-soluble (co)polymer is, before formation of the fracturation fluid:
- either in powder form;
- or in the form of a water-in-oil inverse emulsion;
- or in the form of an aqueous or oil particulate polyphasic suspension.

10. The process according to claim 9, ***characterized* in that** the water-soluble (co)polymer is, before formation of the fracturation fluid, in powder form having a particle size less than 1.5 millimeters, preferably less than 850 micrometers, even more preferably less than 200 micrometers.

11. The process according to one of claims 9 or 10, ***characterized* in that** the water-soluble (co)polymer is, before formation of the fracturation fluid, in the form of an inverse water-in-oil emulsion, the (co)polymer concentration in the emulsion preferably being inclusively between 5 and 60% by weight, preferably between 15 and 40% by weight relative to the weight of the emulsion.

12. The process according to one of claims 9 to 11, ***characterized* in that** the water-soluble (co)polymer is, before formation of the fracturation fluid, in the form of an inverse water-in-oil emulsion containing by weight from 0.01 to 70% of an organic and/or inorganic salt, preferably between 5 and 20% by weight, relative to the weight of the emulsion.

13. The process according to one of claims 9 to 12, ***characterized* in that** the water-soluble (co)polymer is, before formation of the fracturation fluid, in the form of an aqueous particulate polyphasic suspension comprising:
i 15 to 60% by weight of at least one water-soluble (co)polymer in the form of solid particles with average size inclusively between 5 and 500 µm;
ii 15 to 45% by weight of at least one alkali metal salt and/or of at least one alkaline earth metal salt;
iii at least one viscosifying agent other than the water-soluble (co)polymer;
iv at least 10% by weight of water;
said suspension having a Brookfield viscosity inclusively between 500 and 20,000 cps at a temperature of 20°C, and
said suspension having a density inclusively between 1.1 and 2 kg.L⁻¹.

14. The preparation process according to one of claims 9 to 13, ***characterized* in that** the water-soluble (co)polymer is, before formation of the fracturation fluid, in the form of an oil particulate polyphasic suspension comprising:
i 15 to 60% by weight of at least one water-soluble (co)polymer in the form of solid particles with average size inclusively between 5 and 500 µm;
ii at least one viscosifying agent other than the water-soluble (co)polymer;
iii at least 10% by weight of oil;
said suspension having a Brookfield viscosity inclusively between 500 and 20,000 cps at a temperature of 20°C, and
said suspension having a density inclusively between 0.6 and 1.4 kg.L⁻¹.

15. A hydraulic fracturation process for an unconventional underground oil or gas reservoir comprising the preparation of a fracturation fluid according to one of claims 1 to 8, and the injection of said fracturation fluid in an underground formation.

16. A friction reduction process for a fracturation fluid in a hydraulic fracturation operation for unconventional underground oil or gas reservoirs comprising the preparation of a fracturation fluid according to claims 1 to 8, and the injection of said fracturation fluid in an underground formation.
